# EUROPEAN PATENT SPECIFICATION

(11) **EP 1 608 349 B1**
(45) Date of publication and mention of the grant of the patent: **17.10.2007**
(21) Application number: 04757724.2
(22) Date of filing: 17.03.2004
(51) Int. Cl.: A61K 9/70, A61K 31/19, A61K 31/155, A61P 29/00

(54) **ORAL DELIVERY SYSTEM COMPRISING AN ANTIBACTERIAL AND AN ANTI-INFLAMMATORY AGENT**
ORALES ABGABESYSTEM MIT EINEM ANTIBAKTERIELLEN UND EINEM ENTZÜNDUNGSHEMMENDEN MITTEL
SYSTEME D'ADMINISTRATION PAR VOIE ORALE COMPRENANT UN AGENT ANTIBACTERIEN ET UN AGENT ANTI-INFLAMMATOIRE

(30) Priority: 19.03.2003 US 391196
(43) Date of publication of application: 28.12.2005
(73) Proprietor: Dexcel Pharma Technologies Ltd., 91237 Jerusalem (IL)
(72) Inventor: PENHASI, Adel, Holon 58671 (IL); REUVENI, Albert, Jerusalem 93845 (IL); OREN, Dan, Kfar Shmariahu 46910 (IL)
(74) Representative: Minoja, Fabrizio
(86) International application number: PCT/IL2004/000252
(87) International publication number: WO 2004/084873

(56) References cited:
- WO-A-99/04764
- US-A- 5 002 769
- US-A- 6 159 498
- VYAS S P ET AL: "CONTROLLED AND TARGETED DRUG DELIVERY STRATEGIES TOWARDS INTRAPERIODONTAL POCKET DISEASES" JOURNAL OF CLINICAL PHARMACY AND THERAPEUTICS, BLACKWELL SCIENTIFIC PUBLICATION, OXFORD, GB, vol. 25, no. 1, February 2000 (2000-02), pages 21-42, XP000982055 ISSN: 0269-4727

## Description

### FIELD OF THE INVENTION

The present invention relates to oral devices and implants useful in the treatment of dental diseases, more specifically periodontal diseases.

### BACKGROUND OF THE INVENTION

The teeth are principally supported by the alveolar processes of the maxilla and mandible. Collagen fibers course between and insert into the cementum and alveolar bone to hold the teeth in place. The supporting structures of the teeth including the cementum, the periodontal membrane, the bone of the alveolar process, and the gums form the *periodontium.* The ability to chew normally with one's own teeth depends in part upon the health of the periodontium.

Many diseases affect the health of the periodontium and may lead to a loss of alveolar bone and the loosening of teeth. The gingival attachment to the tooth may move apically while the gingiva seemingly remains in place or becomes enlarged. This results in a loose sleeve of diseased gingiva lying against the tooth. The space between this gingiva and the tooth is called a *pocket.* The ultimate result of pocket formation, bone loss and tooth mobility is the loss of teeth.

Periodontal diseases are a very common occurrence affecting, at a conservative estimate, between 70%-90% of the world population and is the major cause of tooth loss in people over 35 years of age. Periodontal disease can be defined as an infection and inflammation of the gingiva or gums that may cause, in severe stages, to loss of the bone that support the teeth. There are varying levels of severity of the disease. The mildest cases are clinically termed *gingivitis,* while the more severe cases are clinically known as *periodontitis.* Gingivitis is an inflammation of the gingiva (or gums) that can be associated with poor oral hygiene and/or the hormonal state of the patient. It is believed that gingivitis, if untreated, will develop into periodontitis. Periodontitis is a bacterial disease that affects the gum tissue, the tooth and the bone that surrounds the tooth. Dental professionals classify the periodontitis into five levels or "types", starting from type I that includes inflammation of the gum tissue with the presence of bleeding but without bone loss and periodontal pockets of 1-3 mm, and ending with type V, which includes severe types characterized by rapid bone loss.

The oral cavity is essentially an aerobic environment, which is perfused by saliva. In contrast, the periodontal microenvironment is more anaerobic and is perfused by a plasma filtrate, known as the "gingival crevicular fluid". The growth of microorganisms within this microenvironment has been shown to be the cause of periodontal disease (Loe, et al, J. Periodontol. 36:177 (1965); Slots, Scan. J. Dent. Res., 85:247 (1977); Socransky, S.S., J. Periodontol. 48:497-504 (1977); Axelsson, P., et al., J. Clin. Periodon. 5:133-151 (1978). Hence, the treatment of the disease is directed toward controlling this growth.

As the periodontal disease becomes more established, the periodontal microenvironment becomes more anaerobic and the flow of gingival crevice fluid increases. A review of periodontal disease, and the methods for its treatment, is provided by Goodson J.M. (In: Medical Applications of Controlled Release, Vol. II, Applications Evaluation (Langer, R.S., et al., Eds.), CRC Press, Inc., Boca Raton, FL (1984), pp.115-138. The increased flow of gingival crevice fluid, which accompanies periodontal disease, has the effect of diluting and removing therapeutic agents placed within the periodontal crevice.

Efforts to treat periodontal disease by agents, e.g. antibacterial agents, provided to the oral cavity are generally ineffective, because the periodontal pocket is substantially inaccessible. On the other hand, systemic administration of antibiotics has only variable success in treating periodontal disease (Genco, R.J., J. Periodontol. 52:545 (1981).

Antibacterial agents such as chlorhexidine and quaternary ammonium salts in the form of mouth rinses have proved to be successful in preventing periodontal disease (Loe et al., J. Periodont. Res. 5:78 (1970). These agents, however, are unable to affect the subgingival flora when administered in this form as they do not penetrate into the pockets which are the result of the disease. Hence, they cannot be used in mouth rinses to treat an established periodontal disease.

The most widely used non-pharmacological approach to date has been mechanical cleaning methods combined with surgery. Although this method has proved to be fairly successful in treating individuals, there is still a high recurrence rate.

Controlled-release pharmaceutical compositions which are capable of being inserted into the periodontal cavity and of slowly releasing an antimicrobial agent have been developed. For example, Goodson J. M. (US Patents No. 4764377 and No. 4892736) discloses the incorporation of tetracycline into nondegradable polymeric fibers which can be wrapped around the tooth and release the antibiotic into the periodontal cavity for several days. The fibers needed to be fastened in place with an adhesive and to be removed at the end of the treatment period.

Dunn, R. L. (US Patent No. 5702716) describes the incorporation of doxycycline into a gel that solidifies in the periodontal pocket. The antibiotic drug is released over several days. The solidified gel must be removed at the end of the treatment.

Degradable polymers and copolymers which have been substantially investigated as potential implant compositions include poly(lactic acid), poly(glycolic acid, poly(lactic acid)-poly(glycolic acid) copolymer), polyamides and copolymers of polyamides and polyesters. However, the biodegradation of some of these polymers and copolymers may require three to five months, which is undesirable in the case of dental therapy.

Suzuki, Y., et. al., (US Patent No. 4,569,837) discloses the use of water-soluble polymeric substances (such as methyl cellulose, gelatin, etc.) as a polymeric matrix for a periodontal implant.

A biodegradable sustained-release composition has been described by Friedman, M. et al., (US Pat. No. 5,023,052 and EP 388220) which is capable of treating periodontal or other diseases. The composition comprises a biodegradable polymeric matrix and a bioactive agent, wherein the polymeric matrix comprises a plasticizing agent and a cross-linked, water-insoluble protein formed from a water soluble protein and a crosslinking agent. The bioactive agent include a variety of pharmaceuticals or biologicals, while in the case of treating a periodontal disease the composition would preferably contain chlorhexidine or a salt thereof. US 5002 769 discloses a biodegradable sustained-release oral delivery system for the treatment of periodontal diseases. The active agent is embedded in a matrix of hydrolysed gelatin cross linked with glutaraldehyde.

The compositions described above have varying efficacy in reducing the bacterial load of the periodontal pocket and in reducing pocket depth. Furthermore, none of the above mentioned formulations are particularly efficacious in imparting anti-inflammatory benefits.

Various studies have been carried out on bone formation using non-steroidal anti-inflammatory drugs (NSAID) as agents affecting bone growth and bone structure. Flurbiprofen (FBP) is an example of an effective NSAID which also exhibits analgesic and anti-pyretic activity. Wechter, W. J. (EP 137668 B1) suggests the use of FBP for the inhibition or prevention of alveolar bone loss or resorption.

Jeffcoat et al (J. Perio. Res. 23:381-385, 1988) were the first investigators who demonstrated the clinical effects of FBP on the progression of periodontal disease. As evidenced by standardized radiography and reduced radiopharmaceutical uptake, treatment with FBP (100 mg/day) for two months increased bone metabolism. A study for 24 months using FBP by Williams et al (J. Dental Res. 70:468,1991) found that the FBP-treated patient group showed reduction in bone loss.

A topical pharmaceutical composition for the treatment of epidermal and muco-epidermal tissues, and designed for immediate release in said tissues, was described by Embro W. J (WO 95/04520). The composition includes chlorhexidine and at least one non steroidal anti-inflammatory agent as the active ingredients. These active ingredients are incorporated into a gel, cream, solution or rinse and are applied topically to sites of epidermal and muco-epidermal inflammation, infection or lesion.

### SUMMARY OF THE INVENTION

The present invention provides a novel pharmaceutical device for the treatment of diseases of the oral cavity, preferably periodontal diseases, with improved patient compliance. The term "treatment" is meant to relate to the act of providing medical aid to a subject in need thereof, and includes acute treatment in an acute phase of a disease, long term or chronic treatment in order to prevent disease progression or recurrence as well as preventive treatment given to subject, e.g. subject who may have some predisposition to develop a disease, in order to prevent disease occurrence in the first place. In the context of the invention it relates to preventing the occurrence of, preventing the development of, or preventing the worsening of diseases of the oral cavity such as periodontal diseases, or for the purposes of ameliorating such condition in such patient, either human or animal. Unless otherwise stated, the term is not limited to any particular length of time, to any therapeutic regime or to any particular dose.

In addition, the terms "device", "system", and "delivery system", are used herein interchangeably and are intended to refer to the oral delivery system of the invention.

As mentioned above, a device for the sustained delivery of water soluble drugs such as chlorhexidine salts, was described in U.S. Pat. No. 5,023,082. It has been surprisingly discovered by the inventors of the present invention that the device of U.S. Pat. No. 5,023,082 is unsuitable for the delivery of hydrophobic agents such as flurbiprofen or for the delivery of combinations of hydrophilic and hydrophobic agents.

Thus, according to a first aspect, the present invention provides an oral delivery device in a solid unit form, that is suitable for implantation into a periodontal pocket and is capable of treating periodontal diseases, where sustained drug release is desired. The pocket might be a natural one, may be caused by a disease state, or may be opened intentionally as part of the treatment (e.g. through surgery). Upon its implantation, the device of the invention is softening, swelling and changing into a soft paste that adheres to the pocket. One of the benefits of this change is the high surface area for reaction concerning the active ingredients. The term. "soft" is meant to describe the state of a material of easily yielding to pressure, easily impressed, molded, or cut; not firm in resisting, malleable, elastic and/or formless.

The device of the invention comprises both a hydrophilic and a hydrophobic drug, a surfactant and a biodegradable or bioerodible pharmaceutically acceptable polymer which provides sustained delivery of at least one of the drugs. Preferably, the device may also comprise at least one of a crosslinking agent, a plasticizing agent a wetting agent, a suspending agent, and a dispersing agent. The polymer, preferably water soluble protein, is compatible with a high percentage of alcohol, a water/alcohol solvent, a hydrophobic NSAID soluble in alcohol, a hydrophilic water soluble antibacterial agent, a crosslinking agent that renders the protein insoluble upon completion of the crosslinking reaction, a plasticizing agent such as glycerin and surface active agents and / or water repelling agents.

More specifically, the invention provides an oral delivery device for the treatment of periodontal diseases, said device being in a solid form and suitable for administration to the periodontal pocket of a patient and comprising:
(i) a biodegradable or bioerodible pharmaceutically acceptable polymer;
(ii) a therapeutically effective amount of at least one antibacterial agent; and
(iii) a therapeutically effective amount of at least one anti-inflammatory agent, the relative weight ratio between the antibacterial agent and the anti-inflammatory agent ranging from about 7:1 to about 1:5 ; and
(iv) a surfactant.

At times, the delivery device may further comprise a cross-linking agent that is present in an amount sufficient to render said polymer water-insoluble, while permitting the release of said active agents mentioned in items (ii) and (iii) above from said delivery system.

Preferably, the device may further comprise a plasticizing agent and at least one of a wetting agent, suspending agent, and dispersing agent.

The polymer is any pharmaceutically acceptable polymer that has the ability to degrade in the oral environment safely and, consequently, disappear. In fact, the polymer is one capable to undergo biodegradation or bioerosion in the oral cavity such that it does not have to be removed from the cavity or the pocket in which it had been inserted.

Preferably, the polymer is a water-soluble protein selected from the group consisting of gelatin, collagen, albumin, an enzyme and fibrinogen, more preferably gelatin and even more preferably hydrolyzed gelatin. In the case of a water-soluble protein, the protein is cross-linked by incubation in the presence of a cross-linking agent and/or crosslinking conditions such as heat, humidity, pressure, radiation, and the same. The extent of crosslinking is such that it renders the protein water-insoluble but still permits the release of the active agents from the composition.

The polymer is preferably present at a concentration of from about 20% to about 70% and the plasticizing agent is present at a concentration of from about 1% to about 15%. In addition, the weight ratio between the bioactive agents and the polymer in the composition, ranges from about 2:1 to about 1:3, while the weight ratio between the plasticizing agent and the polymer is from about 1:10 to about 1:2.

Upon crosslinking, a water-soluble polymer becomes insoluble in water but undergoes biodegradation or bioerosion, thus imparting to the device of the invention the capability to release the active agents in a sustained manner. When the device is placed in the periodontal pocket, it is softening and swelling, thereby adhering to the periodontal pocket More particularly, the device degrades in the oral environment such that it does not have to be removed from the cavity or the pocket in which it has been inserted.

The in-vivo release properties of the delivery device of the invention is such, that once located in a periodontal pocket it gradually releases the anti-inflammatory agent over a period of at least 48 hours, and the antibacterial agent over a period of at least 72 hours, during which the device changes into a soft material.

In a preferred embodiment, the present invention provides a combined flurbiprofen/chlorohexidine di-gluconate oral delivery device, that forms a biodegradable solid unit dosage form which may be employed in the treatment of periodontal diseases or upon periodontal surgery for aiding the healing process and has the release properties described above. More specifically, the present invention provides a solid oral delivery device for periodontal treatment, the device comprising on a weight basis between about 5 to about 25% flurbiprofen, between about 15 to about 35% chlorhexidine di-gluconate, between about 30 to about 50% hydrolyzed gelatin, between about 2 to about 7.5% crosslinking agent, between about 0.5 to about 15% non-ionic surfactant and between about 5 to about 15% plasticizer.

In another aspect, the present invention provides an oral delivery device for the treatment of periodontal diseases, the device being in a solid unit dosage form for administration to the periodontal pocket of a patient and comprising at least two layers, each of which having a matrix comprising a water-soluble polymer crosslinked to yield a water insoluble polymer and an active ingredient. In one of said at least two layers the active agent is an anti-bacterial agent and in another layer of said at least two layers the active agent is an anti-inflammatory agent. In a preferred embodiment of this aspect, the antibacterial agent is chlorhexidine di-gluconate and the anti-inflammatory agent is flurbiprofen.

According to another aspect, the present invention provides an implant device, preferably a periodontal implant device, and the use of said device in treating periodontal or other oral diseases which require prolonged drug release. In a preferred embodiment, the device comprises the flurbiprofen/chlorohexidine di-gluconate combination described above.

In another aspect, the present invention also provides for the use of an oral delivery device as defined above in the preparation of an agent adapted for oral administration for the treatment of a condition of the oral cavity. The invention is further directed to a method of treating periodontal disease by placing the device herein described into a periodontal pocket or implanting the device in an oral pocket or gap before and/or after periodontal surgery, such that the device releases the drugs that are effective in reducing the pocket depth or in aiding the healing process after periodontal surgery.

### BRIEF DESCRIPTION OF THE DRAWINGS

In order to understand the invention and to see how it may be carried out in practice, some preferred embodiments will now be described, by way of nonlimiting example only, with reference to the accompanying drawings, in which:
**Fig. 1** schematically shows a flow chart for the preparation of a bi-layer device.
**Fig. 2** schematically shows a flow chart for the preparation of a tri-layer film.
**Fig. 3** schematically shows a flow chart for the preparation of a precursor solution for the device of the invention (termed "Chip plus").
**Fig. 4** shows a graph that compares the pocket depth reduction, determined by PPD (Probing Pocket Depth), between the device of the invention (termed "Chip plus") that contains chlorhexidine digluconate and flurbiprofen and a device (termed "Periochip) that contains only chlorhexidine digluconate as the active ingredient.
**Fig. 5** shows the in-vivo release profile of CHG and FBP in gingival cervicular fluid from Chip plus and Periochip.
**Fig. 6** shows the in vitro release profile of CHG from Chip plus and Periochip in protease solution.
**Fig. 7** shows the in vitro release profile of CHG from several batches of Chip plus and Periochip in buffer tris+10% AcN.
**Fig. 8** shows the in vitro release profile of FBP from several batches of Chip plus in buffer tris+10% AcN.

### DETAILED DESCRIPTION OF THE INVENTION

The oral delivery device of the present invention may be in the form of films, pellets, granules, or any other convenient shape for the task at hand. Typically, the device is a film formed through the solidification of liquid, precursor solutions. When dried to produce the solid devices (i.e. implants) of the present invention, they may be used as inserts to oral cavities. Most preferably, the implants are in the form of a film and may be used as an insert into periodontal pockets, or as an implant before and/or after periodontal surgery for improving/enhancing/aiding the healing process and allowing the sustained drug delivery at the desired location. The term "film" as used in the present invention denotes a thin sheet or strip of flexible material comprising the system of the invention.

Examples of pharmaceutically acceptable polymers that may be used in the device of the invention include but are not limited to water-soluble proteins, cellulose derivatives such as cellulose acetate, methylcellulose, HPMC (hydroxypropyl methylcellulose), carboxymethyl cellulose (CMC), hydroxyethyl cellulose (HEC), carbomer, PVP (polyvinylpyrrolidone), acacia gum, guar gum, polyvinyl alcohol, polyhydroxyethyl metacrylate, polyhydroxymethyl metacrylate polyacrylic acid, polyacryl amide, polyethylene glycol, starch sodium alginate, polylactic acid, polyglycolic acid, copolymers of polylactic and polyglycolic acid, polyanhydride, polyortho esters and the like.

Proteins have been found useful as the basis for drug delivery systems since their degradation products are harmless amino acids and their biodegradation is facile in many parts of the body. Useful proteins for drug delivery include proteins derived from connective tissue such as collagen and gelatin, and proteins of the albumin class that may be derived from milk, serum, or from vegetable sources, with gelatin and hydrolyzed gelatin being the most preferable. Byco^{™} (a trademark of Croda Colloids, Ltd.) have been found to be the most preferred proteins for use in the device of the present invention.

Proteins tend to be water-soluble. In a water-soluble form the protein is less useful for sustained release of a drug. It is therefore desirable to render the protein insoluble while maintaining its ability to biodegrade through proteases in the body. This insolubilization of the protein may be achieved by making insoluble salts of the protein, insoluble complexes of the protein or most preferably by crosslinking the protein. Since proteins in general contain lysine and arginine residues carrying amino reactive groups and serine, threonine and tyrosine carrying hydroxyl side chains, one preferable and well accepted method of crosslinking proteins is with aldehydes or dialdehydes. Formaldehyde and more preferably glutaraldehyde are well known in the art in methods of crosslinking proteins.

In addition to chemical crosslinking agents, physical means capable of producing crosslinking in proteins may be employed. Examples of such physical means are heat, humidity, pressure or radiation.

The crosslinked protein is rendered water-insoluble but its ability to be degraded by proteases in the body is maintained. The amount of crosslinking can be controlled by the ratio of the crosslinking agent to the protein side groups with which it is to react. The more heavily crosslinked the protein the less soluble it will be and the more slowly it will be biodegraded by protease enzymes. For example, the preferable amount of glutaraldehyde for crosslinking hydrolyzed gelatin has been found to be the amount that is stoicheometric with the amino side chains in the protein. The crosslinked protein can be designed to degrade over a convenient time span, most preferably between about 4 to about 10 days.

The incorporation of the drugs in the delivery system must be uniform so as to keep tight control over the dosing level. If one chooses crosslinked protein because of its delivery, degradation, and non toxic by-product properties, one is faced with a problem of incorporating non water soluble drugs into such a system. When all the components are dissolved in a solution, the mixture of the components upon solidification is considerably more intimate and the control of the drug delivery from the crosslinked protein is much enhanced.

Many drugs that are not soluble to any extent in aqueous solutions are soluble in alcohol solutions. Alcohols which are compatible with the aqueous solutions of the proteins are preferably ethanol, isopropanol and n-propanol, with ethanol being the most preferable. Proteins of low molecular weight and a relatively high proportion of hydrophobic side groups do not precipitate from aqueous solution when a certain proportion of alcohol is added. A preferable protein with regards to this property is hydrolyzed gelatin with an average molecular weight less than 20,000 and most preferably less than 13,000 but higher than 1000. This protein is stable in solutions that contain over 50% ethanol, allowing the incorporation of water insoluble drugs that are soluble in alcohol. When using the hydrolyzed gelatin as the matrix for drug delivery and using glutaraldehyde as the crosslinking agent, it was found that the preferred ethanol to water ratios are between 1.2 and 2.5.

A solid device for insertion into a body crevice needs to be rigid enough to be inserted against a certain amount of back pressure exhibited by the frictional forces on the device when being inserted, but flexible enough so as not to break and flexible enough to conform to the contour of the crevice. Therefore, in order to improve the flexibility of the device of the invention, a plasticizing agent or mixture of such agents is added. The type and the amount of the plasticizer will control the flexibility of the composition. Examples of suitable plasticizers include but are not limited to phthalates such as dimethyl phthalate, dibutyl phthalate, diethyl phthalate; dibutyl sebacate, triethyl citrate, tributyl citrate, acetylated monoglyceride, acetyl tributyl citrate, triacetin, benzyl benzoate, glycol derivatives such as glycerol, polyethylene glycols, propylene glycol butyl and/or glycol esters of fatty acids, refined mineral oils, oleic acid, castor oil, corn oil, camphor and and sugar alcohols such as sorbitol. Preferred plasticizers are sorbitol and glycerin with glycerin being the most preferred plasticizer. The preferred amount of plasticizer is between 1 and 15% and most preferably between 4 and 10%. Further plastic properties of the crosslinked protein are determined by the moisture content of the composition.

The device of the invention may also contain at least one of a wetting agent, solubilizing agent suspending agent, surfactant, and dispersing agent, or a combination thereof, in addition to the plasticizer.

Examples of suitable wetting agents include, but are not limited to, poloxamer, polyoxyethylene ethers, polyoxyethylene sorbitan fatty acid esters (polysorbates), polyoxymethylene stearate, sodium lauryl sulfate, sorbitan fatty acid esters, benzalkonium chloride, polyethoxylated castor oil, docusate sodium.

Examples of suitable suspending agents include but are not limited to, alginic acid, bentonite, carbomer, carboxymethylcellulose, carboxymethylcellulose calcium, hydroxyethylcellulose, hydroxypropyl cellulose, microcrystalline cellulose, colloidal silicon dioxide, dextrin, gelatin, guar gum, xanthan gum, kaolin, magnesium aluminum silicate, maltitol, medium chain triglycerides, methylcellulose, polyoxyethylene sorbitan fatty acid esters (polysorbates), povidone (PVP), propylene glycol alginate, sodium alginate, sorbitan fatty acid esters, and tragacanth.

When preparing the device of the invention, it may be advantageous to include surface active agents, preferably non-ionic surfactants, in order to enhance the solubilization of the components and to stabilize the solutions. The surface active agent may be present in amounts that vary from 0 to about 20% of the delivery device. Examples of suitable surfactants include but are not limited to, anionic surfactants such as polysorbate 80 (Tween 80), anionic emulsifying wax (Crodex A), and sodium lauryl sulfate; cationic, such as cetrimide; nonionic, such as polyoxyethylene sorbitan fatty acid esters (polysorbates) and sorbitan fatty acid esters.

Examples of suitable dispersing agents include but are not limited to, poloxamer, polyoxyethylene sorbitan fatty acid esters (polysorbates) and sorbitan fatty acid esters.

A variety of pharmacological agents may be incorporated into the system of the invention and thus into the devices described herein. The requirements are that the drug is soluble in alcohol so that it is compatible with the precursor solution. According to the present invention, more than one pharmacological agent is incorporated into the device. The agents may be of the same therapeutic category (e.g. two or more anti-inflammatory drugs, or two or more anti-bacterial drugs) or of different therapeutic categories (e.g. one or more anti-bacterial drug and one or more anti-fungal drug, or one or more anti-inflammatory drug and one or more anti-neoplastic drug, or one or more anti-bacterial drug and one or more anti-inflammatory drug). The amount of drugs to be incorporated into the composition depends on the intended therapeutic use and can be determined by one skilled in the art. The drugs can be present in the composition from 1 to 70% (w/w) most preferably between 15 and 60% (w/w).

Suitable drugs which can be administered using the device of the present invention include the following groups:
- anti-inflammatory agents, including steroidal anti-inflammatory agents such as dexamethasone, budesonide, beclomethasone, and hydrocortisone; and non-steroidal anti-inflammatory agents (NSAIDs) such as carprofen, diclofenac, fenbufen, fenclozic acid, fenoprofen, flufenamic acid, flurbiprofen, ibuprofen, indomethacin, indoprofen, ketoprofen, lonazolac, loxoprofen, meclofenamic acid, mefanamic acid, naproxen, proprionic acids, salicylic acids, sulindac, tolmetin, meloxicam, oxicams, piroxicam, tenoxicam, etodolac and oxaprozin; (please confirm that you wish to mention steroidal anti-inflammatory agents)
- anti-bacterial agents such as sulfonamides, phenolics, quaternary ammonium salts, chlorhexidine and salts thereof, antibiotics such as penicillins, cephalosporins, tetracycline, doxycycline, chloramphenicol, and erythromycin;
- anti pain agents for the control of pain from a localized site in the body, for example morphine, codeine, etc.

A particularly preferred combination of pharmacological agents used in the device of the invention will include an anti-inflammatory agent together with an antibacterial agent. An example of a preferred combination includes flurbiprofen and chlorhexidine di-gluconate.

The above mentioned components are mixed in a suitable solvent in any ratio which is capable of producing a liquid composition, which forms, upon drying, a sustained-release composition. The components may be mixed as liquids or as solids to be dissolved in a suitable solvent. Suitable solvents include water, ethanol and water-ethanol mixtures.

The device of the present invention is prepared in solid form by pouring the liquid composition into molds which may then be dried, thus forming the implant of the present invention. It is preferable to use a protein concentration range which results in the formation of a liquid composition having acceptable pourability, and which is capable of releasing the active agents upon its drying into the solid, sustained-release form.

The particular form into which the composition is cast will depend upon its intended use. Thus, for example, if the implant is designed to be used in the treatment of periodontal (or other dental) disease by insertion into the periodontal cavity, then the implant will preferably be cast into a film or film-like sheet. In order to be inserted into a patient's periodontal pocket to treat periodontal or other disease, the implant should preferably be a film having a thickness which ranges from 0.01-1.0 millimeters, and preferably having a thickness of between 0.2 and 0.6 millimeters. In order to be inserted in the periodontal pocket, it is preferable that the implant shape be oval, and/or torpedo (i.e., bullet) and/or rod-like.

Although the width and length of the implant may vary depending upon the size of the periodontal pocket of the recipient patient, it is preferable to use implants having a width of between 1-5 millimeters, and preferably between 3-5 millimeters. It is preferable to employ implants having a length of between 3-10 millimeters, and most preferable to employ implants having a length of between 4-6 millimeters. Typically, an implant of the invention has a length of about 5 mm, a width of about 3.8-4.5 mm and a thickness of about 0.35-0.55 mm.

The implants of the present invention may be individually produced or may be obtained (i.e., cut, ground, etc.) from a larger material (i.e., a block, or film-like sheet).

In addition to its use in the treatment of periodontal disease, the device of present invention may be used in a variety of alternative dental applications. For example, it may be used in the treatment and/or prevention of pericoronitis, to assist with root canal sterilization, to facilitate the healing of gums after tooth extraction and to treat or prevent the problem of "dry socket" or to prevent infection incident to tooth implants.

A further preferred usage of the delivery device is as an adjunct treatment to periodontal surgery where it is inserted into the periodontal pockets both before and after the periodontal surgery.

### EXAMPLES

### Example 1

The liquid precursor compositions in Table 1 below were prepared according to the following procedure: the hydrolyzed gelatin was completely dissolved in chlorhexidine di-gluconate (CHG) solution in water, then the solution of the plasticizer (glycerin) in ethanol, the crosslinking agent (GA), the solution of the surfactant (Tween 80) in ethanol and the solution of the flurbiprofen (FBP) in ethanol were added. Each component was added after completely dissolving the former one. The mixing was carried out by a mechanical stirrer at 500 RPM, at room temperature. The liquid precursors were then poured into a leveled mold to obtain a solid film after drying at 22°C for 48 hours.

**Table 1**

| **Formulation No**. | 500-3 | 500-4 | 500-5 | 500-8 | 500-16 |
|---|---|---|---|---|---|
| **Ingredients** | gr. | | | | |
| BYCO E ⁽¹⁾ | 50.0 | 50.0 | 25.0 | 25.0 | 22.5 |
| GLYCERIN | 5.0 | 5.0 | 5.0 | 5.0 | 2.5 |
| GA(25%)⁽²⁾ | 2.0 | 3.0 | 2.0 | 4.0 | 4.0 |
| TWEEN 80 | 15.0 | 10.0 | 5.0 | 5.0 | 5.0 |
| CHG(20%)⁽³⁾ | 125.0 | 125.0 | 63.5 | 63.5 | 62.5 |
| FBP⁽⁴⁾ | 10.0 | 10.0 | 2.5 | 2.5 | 2.5 |
| P.W⁽⁵⁾ | 43.0 | 52.0 | 30.5 | 28.5 | 91.0 |
| ETHANOL | 40.0 | 35.0 | 15.0 | 15.0 | 10.0 |
| TOTAL | 290.0 | 290.0 | 148.5 | 148.5 | 200.0 |
| GA/BYCO(%) | 1.0 | 1.5 | 2.0 | 4.0 | 4.4 |

| | | | | | |
|---|---|---|---|---|---|
| (1) BYCO E- type of hydrolyzed gelatin (2) GA (25%)- 25% aqueous solution of glutaraldehyde (3) CHG(20%)-20% aqueous solution of chlorhexidine di-gluconate (4) FBP-Flurbiprofen (5) P.W.- pure water | | | | | |

### Example 2

The liquid precursor compositions listed in Table 2 were prepared as in Example 1, except that the solutions of Tween 80 and FBP in ethanol were mixed together before adding to the liquid precursor.

**Table 2**

| **Formulation No.** | 500-9 | 500-10 |
|---|---|---|
| **Ingredients** | gr | gr |
| BYCO E | 25.0 | 22.5 |
| GA(25%) | 4.0 | 4.0 |
| TWEEN 80 | 10.0 | 10.0 |
| CHG(20%) | 63.5 | 63.5 |
| FBP | 2.5 | 5.0 |
| Pure water | 28.5 | 41.0 |
| ETHANOL | 15.0 | 12.5 |
| TOTAL | 148.5 | 158.5 |

### Example 3

The liquid precursor compositions showed in Table 3 were prepared in a manner similar to that described in Example 1.Triethyl citrate was used as plasticizer instead of glycerin.

**Table 3**

| **Formulation** | 555-7 |
|---|---|
| **Ingredients** | gr. |
| BYCO E | 22.5 |
| T.E.C | 4.5 |
| GA(25%) | 2.0 |
| TWEEN 80 | 5.0 |
| CHG(20%) | 62.5 |
| FBP | 5.0 |
| P.W | 30.5 |
| ETHANOL | 15.0 |
| TOTAL | 147 |
| GA/BYCO(%) | 2.2 |

| | |
|---|---|
| T.E.C-triethyl citrate | |

### Example 4

The liquid precursor compositions showed in Table 4 below were prepared according to the following procedure:

Hydrolyzed gelatin was dissolved in pure water using a mechanical stirrer (500 rpm) at room temperature. After complete dissolution, all the remaining components were added to the hydrolyzed gelatin solution. Each component was added after complete dissolving the former one. The crosslinking agent was added in two distinct portions. The liquid precursors were then poured into a leveled mold to obtain a solid film after drying at 22÷C for 48 hours.

**Table 4**

| **Formulation** | 500-17 | 500-18 |
|---|---|---|
| **Ingredients** | | |
| BYCO E | 22.5 | 22.5 |
| GLYCERIN | 2.5 | 2.5 |
| GA(25%) | 4.0 | 4.0 |
| TWEEN 80 | 5.0 | 5.0 |
| CHG(20%) | 62.5 | 62.5 |
| FBP | 2.5 | 2.5 |
| P.W | 91.0 | 91.0 |
| ETHANOL | 10.0 | 10.0 |
| TOTAL | 200.0 | 200.0 |

### Example 5

The liquid precursor compositions showed in Table 5 were prepared. In all formulations tannic acid was used as the crosslinking agent instead of GA. The preparation process was the same as that described in Example 4. Whereas in the liquid precursor of 500-34 tannic acid was added in a powder form, the solutions of tannic acid in pure water were used in both liquid precursor compositions of 500-35 and 500-38.

**Table 5**

| **Formulation** | 500-34 | 500-35 | 500-38 |
|---|---|---|---|
| **Ingredients** | | | |
| BYCO E | 4.5 | 4.5 | 3.6 |
| Glycerin | 0.5 | 0.5 | 0.3 |
| Tannic Acid | 0.3 | 2.0 | 0.5 |
| Tween 80 | 1.0 | 0.5 | 0.7 |
| CHG(20%) | 2.5 | 12.5 | 13.0 |
| FBP | 1.5 | 1.5 | |
| P.W | 13.5 | 23.5 | 10.8 |
| ETHANOL | 2 | 6 | |
| TOTAL | 25.8 | 51.0 | 28.9 |
| TA/BYCO(%) | 0.1 | 0.4 | 0.1 |

### Example 6

The purpose of this experiment was to check the effect of a hydrophobic polymeric component, namely Eudragit L-100-55, in the formulation as an appropriate phase for FBP which is a hydrophobic active agent. The liquid precursor composition showed in Table 6 was used to form the drug delivery solid implants of 500-42. The preparation process was the same as that described for Example 4. An ethanolic solution of Eudragit L-100-55 which included also Tween 80 and FBP was prepared prior to addition to the liquid precursor.

**Table 6**

| **Formulation** | 500-42 |
|---|---|
| **Ingredients** | |
| BYCO E | 6.0 |
| Eudragit L100-55 | 2.0 |
| GA(25%) | 1.2 |
| CHG(20%) | 25.0 |
| FBP | 3.0 |
| TWEEN 80 | 2.0 |
| ETHANOL | 12.0 |
| P.W | 22.8 |
| TOTAL | 74.0 |
| GA/BYCO(%) | 104.2 |

### Example 7

The liquid precursor compositions showed in Table 7 were prepared and used to form the drug delivery solid implants of 500-47 to 500-53. The preparation process was the same as that described for Example 4. In this example the hydrolyzed gelatin was dissolved in a mixture of water and ethanol.

**Table 7**

| **Formulation** | 500-47 | 500-48 | 500-49 | 500-50 | 500-51 | 500-52 | 500-53 |
|---|---|---|---|---|---|---|---|
| **Ingredients** | gr | gr | gr | gr | gr | gr | gr |
| BYCO E | 5 | 5 | 2.5 | 2.5 | 3 | 3 | 3 |
| GLYCERIN | 1 | 1 | 0.5 | 0.5 | 0.4 | 0.8 | 0.8 |
| GA(25%) | 1 | 2 | 1.3 | 1.3 | 1.6 | 1.8 | 1.8 |
| TWEEN 80 | 1.6 | 1.6 | 0 | 0.7 | 0.3 | 0 | 0.3 |
| CHG(20%) | 20 | 20 | 12.5 | 12.5 | 12.5 | 13 | 13.5 |
| FBP | 2.4 | 2.4 | 2 | 1.5 | 1.5 | 1.6 | 1.7 |
| P.W | 46.5 | 48 | 24.4 | 24.4 | 30.4 | 54 | 29.7 |
| ETHANOL | 12.5 | 10.5 | 11.25 | 9.25 | 9.5 | 9.5 | 9.5 |
| TOTAL | 90 | 90.5 | 54.45 | 52.65 | 59.2 | 83.7 | 60.3 |
| GA/BYCO | 5 | 10 | 13 | 13 | 13 | 15 | 15 |

### Example 8

The liquid precursor composition showed in Table 8 was prepared and used to form the drug delivery solid implants of 500-67. The preparation process was the same as that described for Example 4. In this Example the chlorhexidine (CHX) dihydrochloride was used as the antibacterial agent instead of chlorhexidine digluconate.

**Table 8**

| **Formulation** | 500-67 |
|---|---|
| **Ingredients** | gr |
| Byco E | 3.2 |
| GLYCERIN | 0.5 |
| GA(25%) | 0.8 |
| TWEEN 80 | 0.1 |
| CHXHcl | 2.5 |
| FBP | 1.5 |
| P.W | 10.8 |
| ETHANOL | 9.2 |
| TOTAL | 28.6 |
| GA/BYCO | 6.25 |

### Example 9

The effect of GA/hydrolyzed gelatin ratio was determined by preparing the liquid precursor compositions shown in Table 9.

**Table 9**

| **Formulation** | 500-72 | 500-73 | 500-74 | 500-75 |
|---|---|---|---|---|
| **Ingredients** | gr | gr | gr | gr |
| BYCO M | 5.7 | 5.4 | 5.2 | 5.3 |
| GLYCERIN | 0.9 | 0.8 | 0.8 | 0.8 |
| GA(25%) | 1.8 | 2.2 | 2.8 | 2.4 |
| TWEEN 80 | 0.2 | 0.2 | 0.2 | 0.2 |
| CHG(20%) | 24.2 | 23.3 | 23.3 | 23.3 |
| FBP | 2.7 | 2.6 | 2.6 | 2.6 |
| P.W | 18.4 | 19.5 | 17.2 | 19.5 |
| ETHANOL | 8.8 | 9.6 | 9.2 | 12.1 |
| TOTAL | 62.7 | 63.6 | 61.3 | 66.2 |
| GA/BYCO | 7.89 | 10.19 | 13.46 | 11.32 |

### Example 10: Bilayer film: A lamination film forming process

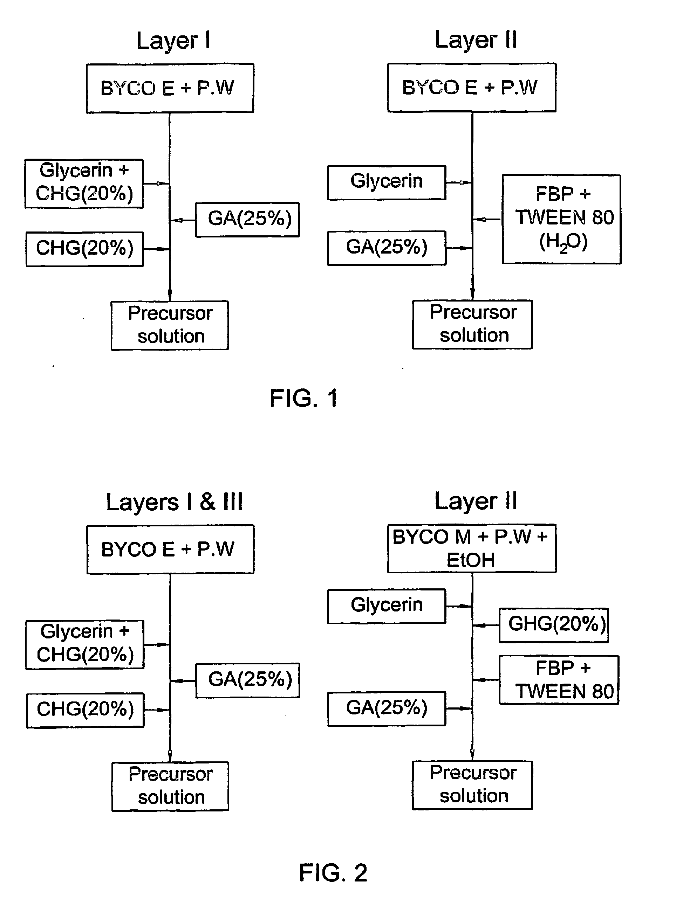

A bi-layer laminated film forming process has been provided where each layer contains either CHG or FBP. The formulations of both layers are shown in Table 10. The liquid precursor composition of each layer was prepared according to the scheme in **Fig. 1**. The liquid precursor of layer I was first poured into a leveled mold to obtain a solid film after drying at 22°C for 48 hours. Then the liquid precursor II was poured onto the solid film of the layer I to obtain a bi-layer laminated solid film after additional drying at 22°C for 48 hours.

**Table 10**

| **Formulation** | 500-94-1 | 500-94-II | 500-95-1 | 500-95-II | 500-96-1 | 500-96-II |
|---|---|---|---|---|---|---|
| **Ingredients** | | | | | | |
| BYCO E | 2.4 | 1.2 | 2.4 | 1.2 | 2.4 | 1.2 |
| GLYCERIN | 0.36 | 0.18 | 0.36 | 0.18 | 0.36 | 0.18 |
| GA(25%) | 1.3 | 0.6 | 1.3 | 0.6 | 1.3 | 0 |
| TWEEN 80 | 1 | 0.15 | 0 | 0.15 | 0 | 0.15 |
| CHG(20%) | 15 | 0 | 15 | 0 | 15 | 0 |
| FBP | 0 | 1.7 | 0 | 1.7 | 0 | 1.7 |
| P.W | 20.2 | 24.9 | 20.2 | 24.9 | 20.2 | 24.9 |
| ETANOL | 0 | 0 | 0 | 0 | 0 | 0 |
| TOTAL | 40.26 | 28.73 | 39.26 | 28.73 | 39.26 | 28.13 |
| GA/BYCO | 13.54 | 12.50 | 13.54 | 12.50 | 13.54 | 0.00 |

### Example 11

A tri-layer laminated film forming process has been developed where the outer layers (layer I and layer II) contain CHG whereas the intermediate layer contains FBP. The preparation process of the liquid processor of the outer layers was the same. The formulations of all layers are shown in Table 11. The preparation process of the laminated film of layer I and II was performed as described in Scheme 1. The layer III was then laminated onto solid film of bi-layer (I and II) on the side of the layer II, by pouring the liquid precursor of layer III and subsequently drying at 22÷C for 48 hours. The entire process is described in **Fig. 2**.

**Table 11: Three layered film**

| **Formulation** | 500-109-1 | 500-109-II | 500-109-III |
|---|---|---|---|
| **Ingredients** | gr | gr | gr |
| BYCOE | 1 | 1 | 1 |
| GLYCERIN | 0.15 | 0.15 | 0.15 |
| GA(25%) | 0.32 | 0.32 | 0.32 |
| TWEEN 80 | 0 | 0.1 | 0 |
| CHG(20%) | 2.74 | 7.4 | 2.74 |
| FBP | 0 | 1.44 | 0 |
| P.W | 14.76 | 9 | 14.76 |
| ETHANOL | 0 | 5.2 | 0 |
| TOTAL | 18.97 | 24.61 | 18.97 |
| GA/BYCO | 8.00 | 8.00 | 8.00 |

### Example 12: Scaled up production process

The production of the implant device of the invention was scaled up to 2Kg and 6Kg. The production process was carried out as follows:

Hydrolyzed gelatin was dissolved in an aqueous/alcohol solvent and the solution was mixed for 45min. Glycerin was added and the mixing was continued for 15 min. Chlorohexidine digluconate solution-20% was added and mixing was continued for 15 min. Glutaraldehyde solution 10% was added and the solution was mixed for 15 min.

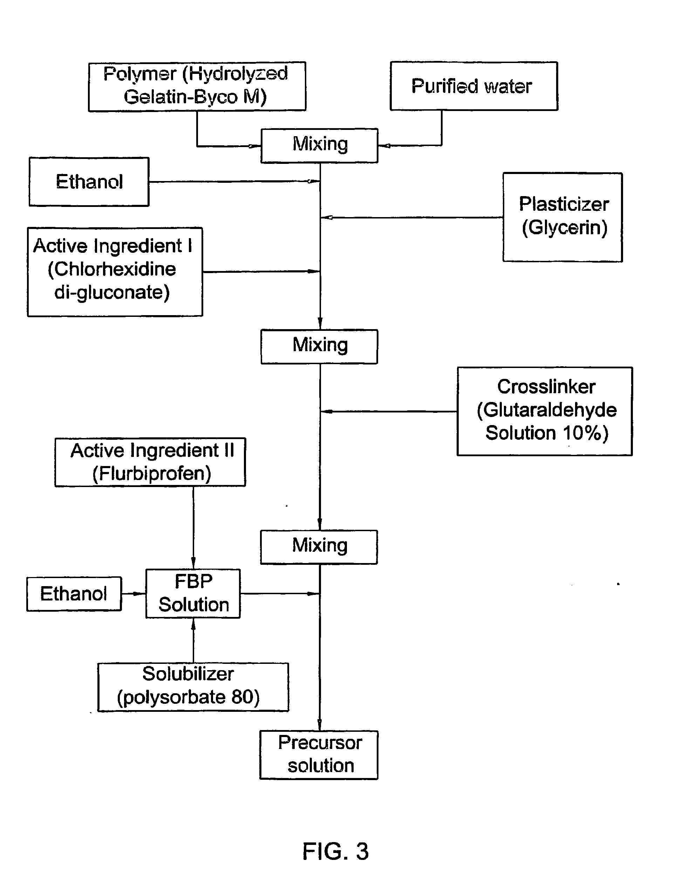

Flurbiprofen in an alcoholic solubilizer mixture was added and the mixing of the resulting mixture was continued for further 15 min. The formulations of the precursors are shown in Tables 12I and 12II for the scale up to 2Kg and 6Kg respectively. The preparation process of the precursor solution is shown in **Fig. 3**.

**Table 12I: scale-up to 2 kg**

| **Formulation** | | |
|---|---|---|
| | W(g) | W(%) |
| BYCO | 180.0 | 9.2 |
| Glycerin | 27.0 | 1.4 |
| GA (25%) | 56.0 | 2.9 |
| Tween 80 | 5.0 | 0.3 |
| CHG(20%) | 765.0 | 39.0 |
| FBP | 85.0 | 4.3 |
| EtOH | 309.0 | 15.8 |
| H₂O | 533.0 | 27.2 |
| TOTAL | 1960.0 | 100.0 |
| GA/BYCO | 0.078 | 0.078 |
| glycerin/Byco | 0.150 | 0.150 |

**Table 12 II: Scale up to about 6 Kg**

| **Formulation** | | |
|---|---|---|
| | W(gr) | W(%) |
| BYCO | 480.0 | 8.4 |
| Glycerin | 82.0 | 1.4 |
| GA (25%) | 150.0 | 2.6 |
| Tween 80 | 13.0 | 0.2 |
| CHG(20%) | 2040.0 | 35.7 |
| FBP | 230.0 | 4.0 |
| EtOH | 940.0 | 14.7 |
| H₂O | 1785.0 | 33.0 |
| TOTAL | 5720.0 | 100.0 |
| GA/BYCO | 0.078 | 0.078 |
| glycerin/Byco | 0.171 | 0.171 |

### Example 13: In-Vivo efficacy trial

The efficacy of the device of the invention was indicated by probing pocket depths (PPD) for all teeth using a standard 15mm, North Carolina periodontal probe. The pocket depth was recorded before and after inserting the device and the % change (reduction) was calculated. At the target sites scaling and root planing was followed by placement of a FBP/CHG implant of the invention, consisting of 2.5 mg of chlorhexidine di-gluconate and 1.5 mg flurbiprofen formulated in a biodegradable cross-linked fish gelatin matrix. The probing pocket depth (mm) and reduction in pocket depth upon the treatment (%) are shown in Table 13.

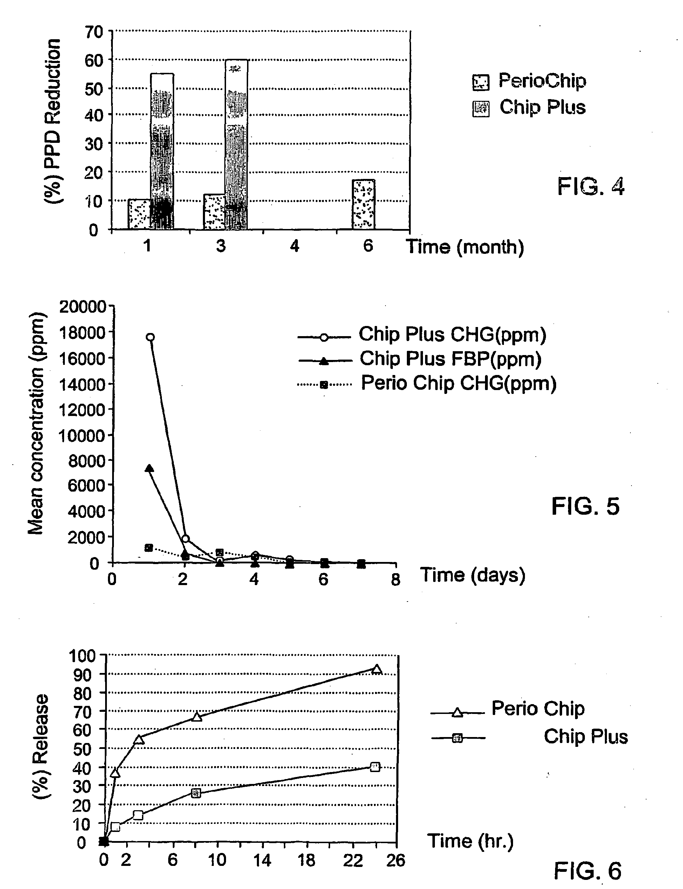

A comparison test was carried out between the efficacy of the device of the present invention, termed hereinafter *Chip Plus,* and comprising 2.5 mg of chlorhexidine digluconate and 1.5 mg flurbiprofen and that of a device containing only 2.5 mg chlorhexidine digluconate, termed hereinafter *PerioChip* and prepared according to the procedure described in US 5,023,082. The results are demonstrated in **Fig. 4**. The data show that a significant reduction in pocket depth is obtained upon using Chip Plus than PerioChip during a comparable period of treatment. In fact it was not clinically necessary to use Chip Plus over 3 months of treatment, while the pockets that received PerioChip, remained >5 mm in depth after 3 and 6 months of treatment.

### Example 14: The in vivo release profile

The objective of this experiment was to determine the release profile of both CHG and FBP in periodontal pocket in-vivo. The Chip Plus was inserted into a periodontal pocket of 5 mm or more in 5 volunteers (12 pockets). The gingival cervicular fluid (GCF) was collected by inserting Periopaer Gingival collection Strips (HARCO Company Inc.) into the pocket for a predetermined time interval. The GCF sampling was carried out prior to Chip insertion for a zero time sample and at one hour, 24 hours, 2 days, 3 days, 6 days, 8 days, and 10 days. The paper strip was inserted into the pocket for thirty seconds. The volume of GCF in the paper strip was measured using a Periotron 6000 (Siemans Company Inc.) The Periotron 6000 was calibrated prior to and during trial. The concentration of both FBP as well as CHG was determined by extraction from the paper strip and determination in an HPLC analysis developed for this project. A comparison between the release profile of Chip Plus and PerioChip for CHG release has been drawn. The results of the In vivo release of both FBP as well as CHG (for both PerioChip and Chip Plus) are shown in Table 14 and Fig. 5.

**Table 13: In Vivo release of FBP and CHG**

| | PerioChip | | ChipPlus | Chip Plus |
|---|---|---|---|---|
| Time(hr.) | CHG (ppm) | Time(hr.) | CHG(ppm) | FBP (ppm) |
| 1 | 1162 | 1 | 8948 | 7382 |
| 24 | 515.8 | 24 | 1816 | 877 |
| 48 | 841 | 48 | 186 | 59 |
| 72 | 482 | 72 | 125 | 36 |
| 144 | 127.1 | 96 | 30 | |
| 192 | 103 | 168 | 7 | |
| 240 | 69.4 | | | |

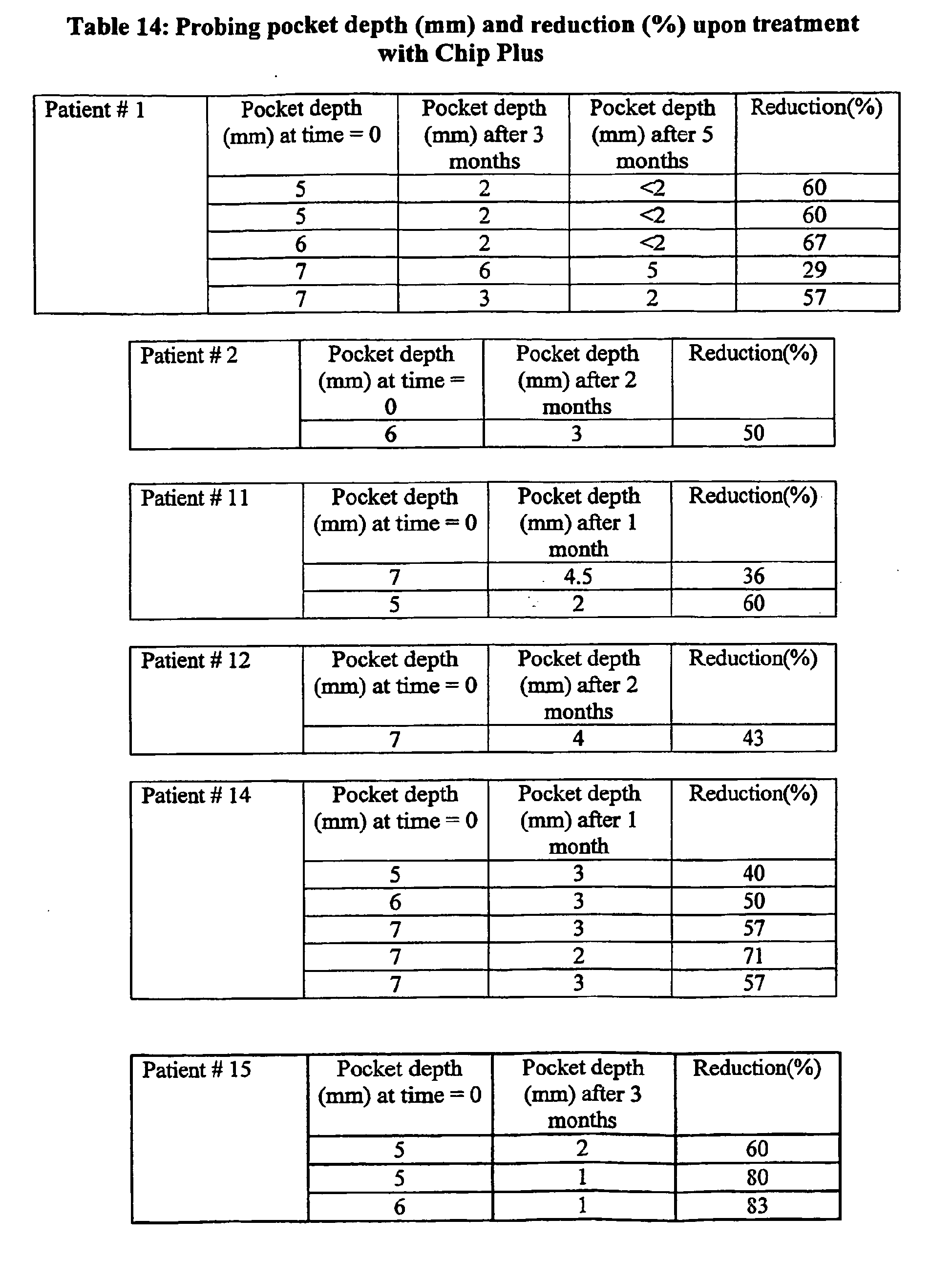

### Example 15: The in vitro release-protease method for CHG

Chip Plus and PerioChip were placed into a test tube with a protease solution of fixed activity and incubated for one, three, eight and twenty four hours. At each time point the protease solution is removed for analysis and fresh protease solution is added to the Chip. CHG released from the chip is then determined in the sample of each time point by extraction and spectrophotometric method. The results of CHG release from Chip Plus as compared to that of PerioChip are shown in **Fig.6**.

### Example 16: In vitro release of CHG -Dissolution method

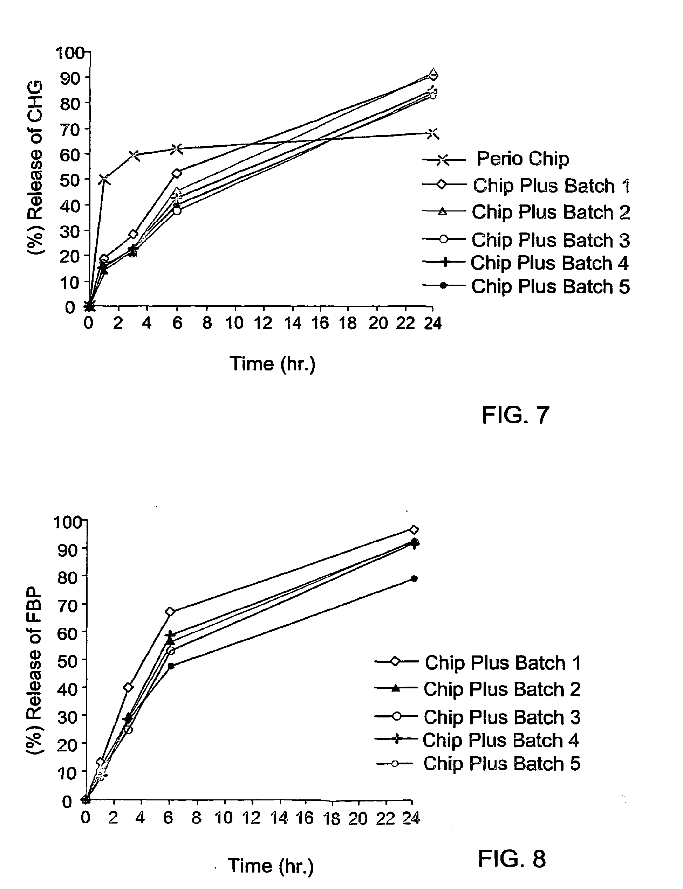

The release of CHG from both Chip Plus and PerioChip was determined using a dissolution method at 37÷C. The dissolution test was performed in a 900 ml water containing 2% Tween 80. 5 chips were placed in each vessel containing 900 ml of the dissolution medium. The basket speed was set at 100 rpm. The amount of CHG released from the chips at each point of time was determined using an HPLC method. The results are summarized in Table 15 and shown in **Fig.7.**

**Table 15: CHG- In Vitro release of Chip Plus from different batches, compared to PerioChip**

| | Periochip | Chip Plus, batch1 | Chip Plus, batch2 | Chip Plus, batch3 | Chip Plus, batch4 | Chip Plus, batch5 |
|---|---|---|---|---|---|---|
| Time(hr.) | CHG(%) | CHG(%) | CHG(%) | CHG(%) | CHG(%) | CHG(%) |
| 0 | 0 | 0 | 0 | 0 | 0 | 0 |
| 1 | 50.2 | 16.9 | 15.6 | 14.9 | 14.4 | 18.9 |
| 3 | 59.5 | 20.8 | 22.8 | 21.2 | 22.1 | 28.6 |
| 6 | 62.3 | 38.1 | 43 | 39.9 | 46.1 | 52.6 |
| 24 | 68.9 | 85 | 84.7 | 83.7 | 92.9 | 91.4 |

### Example 16: In vitro release of FBP

The release of FBP from Chip Plus was determined using a dissolution method at 37°C. The dissolution test was performed in a 900 ml water containing 2% Tween 80. Five chips were placed in each vessel containing 900 ml of the dissolution medium. The basket speed was set at 100 rpm. The amount of FBP released from the chips at each point of time was determined using an HPLC method. The results of release profile from different batches are summarized in Table 16 and shown in **Fig. 8.**

**Table 16: In vitro release of Flurbiprofen from several batches of Chip Plus**

| | Batch 1 | Batch 2 | Batch 3 | Batch 4 | Batch 5 |
|---|---|---|---|---|---|
| Time(hr.) | FBP(%) | FBP(%) | FBP(%) | FBP(%) | FBP(%) |
| 0 | 0 | 0 | 0 | 0 | 0 |
| 1 | 9.7 | 8.1 | 8.2 | 11.3 | 13.2 |
| 3 | 25 | 28.7 | 28.1 | 29.8 | 40.1 |
| 6 | 53.5 | 58.9 | 47.9 | 57 | 67.3 |
| 24 | 92.7 | 92.2 | 79.6 | 93.1 | 97.2 |

## Claims

1. An oral delivery device for the treatment of periodontal disease, said device being in a solid unit dosage form for administration to a patient, comprising:
(i) a biodegradable or bioerodible pharmaceutically acceptable polymer;
(ii) a therapeutically effective amount of at least one antibacterial agent;
(iii) a therapeutically effective amount of at least one anti-inflammatory agent, the relative weight ratio between the antibacterial agent and the anti-inflammatory agent ranging from about 7:1 to about 1:5; and
(iv) a surfactant.

2. An oral delivery device for the treatment of periodontal diseases, said device being in a solid unit dosage form for administration to a patient and comprising at least two layers, each of which having a matrix comprising a biodegradable or bioerodible pharmaceutically acceptable polymer and an active ingredient, in one of said at least two layers said active agent being an anti-bacterial agent and in another layer of said at least two layers said active agent being an anti-inflammatory agent, said system once in the oral cavity gradually releases the active agents from said layers.

3. The device of Claim 1 or claim 2, further comprising a cross-linking agent present in an amount sufficient to render said polymer water-insoluble, while permitting the release of said active agents from said delivery system.

4. *The device of claim 1, wherein the surfactant is selected from anionic surfactants, cationic surfactants and non-ionic surfactants.*

5. The device of claim 4 wherein the non-ionic surfactants is selected from polyoxyethylene sorbitan fatty acid esters (polysorbates) and sorbitan fatty acid esters.

6. The device of Claims 1, 2, 3, 4 or 5 further comprising a plasticizing agent and at least one of a wetting agent, a suspending agent and a dispersing agent.

7. The device of any one of Claims 1 to 6 adapted for administration to a periodontal pocket having in-vivo release properties that yield to reducing the periodontal pocket depth of a patient.

8. The device of Claim 7, being such that it biodegrades in the periodontal pocket thereby becoming soft and adhering to the periodontal pocket, and wherein once located in a periodontal pocket gradually releases said anti-inflammatory agent over a period of at least about 48 hours, and said antibacterial agent over a period of at least about 72 hours, during which said system changes into a soft material.

9. The device of any one of Claims 1 to 8 wherein said polymer is selected from water-soluble protein, cellulose or cellulose derivative, starch or starch derivative, glyceryl monostearate, carbomer, PVP (polyvinylpyrrolidone), gum, acacia gum, guar gum, polyvinyl alcohol, polyhydroxyethyl metacrylate, polyhydroxymethyl metacrylate polyacrylic acid, polyacryl amide, polyethylene glycols, polylactic acid, polyglycolic acid, copolymers of polylactic acid and polyglycolic acid, polyanhydrides and polyorthoesters.

10. The device of any one of Claims 1 to 9 wherein said anti-inflammatory agent is a non-steroidal anti-inflammatory agent (NSAID) wherein said non-steroidal anti- inflammatory agent is selected from the group consisting of flurbiprofen, carprofen, diclofenac, fenbufen, fenclozic acid, fenoprofen, flufenamic acid, ibuprofen, indomethacin, indoprofen, ketoprofen, lonazolac, loxoprofen, meclofenamic acid, mefanamic acid, naproxen, proprionic acids, salicylic acids, sulindac, tolmetin, meloxicam, oxicams, piroxicam, tenoxicam, etodolac and oxaprozin.

11. The device of any one of Claims 1 to 10 in the form of a film.

12. The device of Claim 11 wherein said water-soluble protein is selected from the group consisting of gelatin, collagen, albumin, an enzyme and fibrinogen.

13. The device of any one of Claims 1 to 12 wherein said polymer is cross-linked by a curing process in the presence of a cross-linking agent, wherein said curing process is selected from the group consisting of heat, humidity, pressure, radiation, and the vapors of a cross-linking agent and wherein the cross-linking renders said protein water-insoluble, while permitting gradual release of the active agents from said system.

14. The device of Claim 6 wherein said plasticizing agent is selected from glycol derivatives, phthalates, citrate derivatives, benzoates, butyl or glycol esters of fatty acids, refined mineral oils, camphor, oleic acid, castor oil, corn oil and sugar alcohols.

15. The device of any one of Claims 1 to 14 wherein said antibacterial agent is selected from the group consisting of penicillin, cephalosporin, tetracycline, oxytetracycline, chlortetracycline, metronidazole, chloramphenicol, streptomycin, neomycin, a sulfonamide, a phenolic compound, a mercurial, a quarternary ammonium compound, doxycycline and chlorhexidine or salts thereof.

16. An oral delivery device according to claim 1 for the treatment of periodontal diseases, said device being in a single solid unit dosage form for administration to the periodontal pocket of a patient for changing in said pocket within a soft form, the system comprising on a weight basis, between about 5 to about 25% flurbiprofen, between about 15 to about 35% chlorhexidine di-gluconate, between about 30 to about 50% hydrolyzed gelatin, between about 2 to about 7.5% crosslinking agent, between about 0.5 to about 15% surfactant and between about 5 to about 15% plasticizer.

17. A periodontal implant comprising the device of any one of Claims 1 to 16.

18. Use of:
(i) a biodegradable or bioerodible pharmaceutically acceptable polymer;
(ii) a therapeutically effective amount of at least one antibacterial agent;
(iii) a therapeutically effective amount of at least one anti-inflammatory agent, the relative weight ratio between the antibacterial agent and the anti-inflammatory agent ranging from about 7:1 to about 1:5;
(iv) a surfactant;
for the preparation of a pharmaceutical composition for use in the treatment of dental conditions.

## Patentansprüche

1. Ein orales Abgabgesystem für die Behandlung von periodontaler Krankheit, wobei das System in einer festen Darreichungsform zur Verabreichung an einem Patienten vorliegt, umfassend:
(i) ein biologisch abbaubares oder bioerodierbares pharmazeutisch annehmbares Polymer;
(ii) eine therapeutisch wirkungsvolle Menge von mindestens einem antibakteriell wirkenden Mittel;
(iii) eine therapeutische wirkungsvolle Menge von mindestens einem entzündungshemmenden Mittel, wobei das relative Gewichtsverhältnis zwischen dem antibakteriell wirkenden Mittel und dem entzündungshemmenden Mittel von ungefähr 7 : 1 bis ungefähr 1 : 5 beträgt; und
(iv) ein obenflächenaktives Mittel.

2. Ein orales Abgabesystem für die Behandlung von periodontalen Krankheiten, wobei das System in einer festen Darreichungsform zur Verabreichung an einem Patienten vorliegt und mindestens zwei Schichten umfasst, wobei jede eine Matrix aufweist, welche ein biologisch abbaubares oder bioerodierbares pharmazeutisch annehmbares Polymer und einen aktiven Wirkstoff umfasst, wobei in einer von diesen mindestens zwei Schichten der aktive Wirkstoff ein antibakteriell wirkendes Mittel ist und in der anderen Schicht von diesen mindestens zwei Schichten der aktive Wirkstoff ein entzündungshemmendes Mittel ist, wobei das System sobald es in den Mundraum gelangt, graduell die aktiven Wirkstoffe aus diesen Schichten freisetzt.

3. Das System nach Anspruch 1 oder Anspruch 2, welches darüber hinaus ein Vernetzungsmittel umfasst, welches in einer Menge vorhanden ist, die ausreichend ist, um das Polymer wasserunlöslich zu machen, während die Freisetzung von den aktiven Wirkstoffen aus dem Abgabesystem erlaubt wird.

4. Das System nach Anspruch 1, worin das oberflächenaktive Mittel ausgewählt ist aus anionischen oberflächenaktiven Mitteln, kationischen oberflächenaktiven Mitteln und nichtionischen oberflächenaktiven Mitteln.

5. Das System nach Anspruch 4, worin die nichtionischen oberflächenaktiven Mittel ausgewählt sind aus Polyoxyethylen-Sorbitanfettsäureestern (Polysorbat) und Sorbitan-Fettsäureestern.

6. Das System nach den Ansprüchen 1, 2, 3, 4 oder 5, welches darüber hinaus einen Weichmacher oder mindestens ein Mittel aus einem Benetzungsmittel, einem Stellmittel und einem Dispergiermittel umfasst.

7. Das System nach einem der Ansprüche 1 bis 6, welches angepasst ist zur Verabreichung in eine periodontale Tasche mit in-vivo Freisetzungseigenschaften, welche darauf abzielen, die Tiefe einer periodontalen Tasche eines Patienten zu reduzieren.

8. Das System nach Anspruch 7, welches in solch einer Form vorliegt, dass es in der periodontalen Tasche biologisch abgebaut wird, wobei es **dadurch** weich wird und an der periodontalen Tasche haftet, und worin es, wenn es einmal in einer periodontalen Tasche gegeben wurde graduell das entzündungshemmende Mittel über einen Zeitraum von mindestens ungefähr 48 Stunden freisetzt und das antibakteriellwirkende Mittel über einen Zeitraum von mindestens ungefähr 72 Stunden freisetzt, während dessen sich das System in ein weiches Material umwandelt.

9. Das System nach einen der Ansprüche 1 bis 8, worin das Polymer ausgewählt ist aus wasserlöslichem Protein, Cellulose oder Cellulose-Derivat, Stärke oder Stärke-Derivat, Gylcerylmonostearat, Carbomer, PVP (Polyvinylpyrrolidon), Gummi, Akaziengummi, Guargummi, Polyvinylalkohol, Polyhydroxyethylmethacrylat, Polyhydroxymethylmethacrylatpolyacrylsäure, Polyacrylamid, Polyethylenglykolen, Polyessigsäure, Polyglykolsäure, Copolymeren von Polyessigsäure und Polyglykolsäure, Polyanhydriden und Polyorthoestern.

10. Das System nach einen der Ansprüche 1 bis 9, worin das entzündungshemmende Mittel ein nichtsteroides entzündungshemmendes Mittel (NSAID) ist, worin das nichtsteroide antientzündungshemmende Mittel ausgewählt ist aus der Gruppe, bestehend aus Flurbiprofen, Carprofen, Diclofenac Fenbufen Fenclozin-Säure, Fenoprofen, Flufenamin-Säure, Ibuprofen, Indomethacin, Indoprofen, Ketoprofen, Lonazolac, Loxoprofen, Meclofenamin-Säure, Mefanamin-Säure, Naproxen, Propionsäuren, Salicylsäuren, Sulindac, Tolmetin, Meloxicam, Oxicame, Piroxicam, Tenoxicam, Etodolac und Oxaprozin.

11. Das System nach einen der Ansprüche 1 bis 10 in der Form eines Filmes.

12. Das System nach Anspruch 1 1 , worin das wasserlösliche Protein ausgewählt ist aus der Gruppe, bestehend aus Gelatine, Collagen, Albumin, einem Enzym und Fibrinogen.

13. Das System nach einen der Ansprüche 1 bis 12, worin das Polymer durch einen Vernetzungsprozess in der Gegenwart eines Vernetzungsmittels vernetzt wird, worin der Vernetzungsprozess ausgewählt ist aus der Gruppe, bestehend aus Wärme, Feuchtigkeit, Druck, Bestrahlung und den Dämpfen eines Vernetzungsmittels und worin die Vernetzung das Protein wasserunlöslich macht, während die graduelle Freisetzung der aktiven Wirkstoffe aus dem System erlaubt wird.

14. Das System nach Anspruch 6, worin der Weichmacher ausgewählt ist aus Glykolderivaten, Phthalaten, Citratderivaten, Benzoaten, Butyl- oder Glykolestern von Fettsäuren, hochraffinierten Mineralölen, Kampfer, Ölsäure, Castoröl, Maiskeimöl und Zuckeralkoholen.

15. Das System nach einen der Ansprüche 1 bis 14, worin das anitbakteriell wirkende Mittel ausgewählt ist aus der Gruppe, bestehend aus Penicillin, Cephalosporin, Tetracyclin, Oxytetracyclin, Chlortetracyclin, Metronidazol, Chloramphenicol, Streptomycin, Neomycin, einem Sulfonamid, einer phenolischen Verbindung, einer quecksilberhaltigen Verbindung, eine quartären Ammonium-Verbindung, Doxycylcin und Chlorhexidin oder Salze davon.

16. Ein orales Abgabesystem nach Anspruch 1 für die Behandlung von periodontalen Krankheiten, wobei das System in einer festen Dosierungseinheitsform zur Verabreichung in die periodontale Tasche eines Patienten zur Umwandlung in eine weiche Form in dieser Tasche vorliegt, wobei das System auf Gewichtsbasis umfasst zwischen ungefähr 5 bis ungefähr 25 % Flurbiprofen, zwischen ungefähr 15 bis ungefähr 35 % Chlorhexidin-di-gluconat, zwischen ungefähr 30 bis ungefähr 50 % hydrolisierte Gelatine, zwischen ungefähr 2 bis ungefähr 7,5 % Vernetzungsmittel, zwischen ungefähr 0,5 bis ungefähr 15 % Tensid und zwischen ungefähr 5 bis ungefähr 15 % Weichmacher.

17. Ein periodontales Implantat, umfassend das System gemäß einen der Ansprüche 1 bis 16.

18. Verwendung von
(i) einem biologisch abbaubaren oder bioerodierbaren pharmazeutisch annehmbaren Polymer;
(ii) einer therapeutisch wirkungsvollem Menge mindestens eines antibakteriell wirkenden Mittels;
(iii) einer therapeutisch wirkungsvollen Menge von mindestens einem entzündungshemmenden Mittel, wobei das relative Gewichtsverhältnis zwischen dem antibakteriell wirkenden Mittel und dem entzündungshemmenden Mittel zwischen ungefähr 7 : 1 bis ungefähr 1 : 5 beträgt;
(iv) eines oberflächenaktiven Mittels;
für die Herstellung einer pharmazeutischen Zusammensetzung für die Verwendung in der Behandlung von dentalen Zuständen.

## Revendications

1. Dispositif à libération orale pour le traitement de maladie parodontale, ledit dispositif étant sous forme de dose unitaire solide pour une administration à un patient, comprenant:
(i) un polymère biodégradable ou bioérodable acceptable sur le plan pharmaceutique ;
(ii) une quantité efficace sur le plan thérapeutique d'au moins un agent antibactérien ;
(iii) une quantité efficace sur le plan thérapeutique d'au moins un agent anti-inflammatoire, le rapport de poids relatif entre l'agent antibactérien et l'agent anti-inflammatoire allant de 7:1 environ à 1:5 environ; et
(iv) un tensioactif.

2. Dispositif à libération orale pour le traitement de maladies parodontales, ledit dispositif étant sous forme de dose unitaire solide pour une administration à un patient, et comprenant au moins deux couches, chacune d'entre elles ayant une matrice comprenant un polymère biodégradable ou bioérodable acceptable sur le plan pharmaceutique et un ingrédient actif, dans l'une desdites au moins deux couches ledit agent actif étant un agent antibactérien et dans une autre couche desdites au moins deux couches ledit agent actif étant un agent anti-inflammatoire, ledit système, une fois dans la cavité orale, libère graduellement les agents actifs à partir desdites couches.

3. Dispositif selon la revendication 1 ou 2 comprenant en outre un agent de réticulation présent en une quantité suffisante pour rendre ledit polymère insoluble dans l'eau, tout en permettant la libération desdits agents actifs à partir dudit système de libération.

4. Dispositif selon la revendication 1, dans lequel le tensioactif est sélectionné parmi les tensioactifs anioniques, les tensioactifs cationiques et les tensioactifs non-ioniques.

5. Dispositif selon la revendication 4, dans lequel le tensioactif non-ionique est sélectionné parmi les esters d'acides gras de polyoxyéthylène sorbitan (polysorbates) et les esters d'acides gras de sorbitan.

6. Dispositif selon les revendications 1, 2, 3, 4 ou 5, comprenant en outre un agent plastifiant et au moins un agent mouillant, un agent de suspension et un agent dispersant.

7. Dispositif selon l'une quelconque des revendications 1 à 6, adapté pour une administration à une poche parodontale ayant des propriétés de libération in vivo qui conduisent à réduire la profondeur de la poche parodontale chez un patient.

8. Dispositif selon la revendication 7, qui est tel qu'il se biodégrade dans la poche parodontale, devenant ainsi mou et adhérant à la poche parodontale, et qui, une fois placé dans la poche parodontale libère graduellement ledit agent anti-inflammatoire pendant une période d'au moins 48 heures et ledit agent antibactérien pendant une période d'au moins 72 heures, durant laquelle ledit système se transforme en matériau mou.

9. Dispositif selon l'une quelconque des revendications 1 à 8, dans lequel ledit polymère est choisi parmi une protéine soluble dans l'eau, la cellulose ou un dérivé de cellulose, l'amidon ou un dérivé d'amidon, le glycéryl monostéarate, le carbomer, la PVP (polyvinylpyrrolidone), une gomme, la gomme d'acacia, la gomme de guar, un alcool polyvinyl, le métacrylate de polyhydroxyéthyl, l'acide polyacrylique de polyhydroxyméthyl métacrylate, le polyacryl amide, les polyéthylène glycols, l'acide polylactique, l'acide polyglycolique, les copolymères d'acide polylactique et d'acide polyglycolique, les polyanhydrides et les polyorthoesters.

10. Dispositif selon l'une quelconque des revendications 1 à 9, dans lequel ledit agent anti-inflammatoire est un agent anti-inflammatoire non stéroïdien (NSAID), ledit agent anti-inflammatoire non stéroïdien étant choisi dans le groupe constitué de flurbiprofène, carprofène, diclofénac, fenbufène, acide fenclozique, fénoprofène, acide flufénamique, ibuprofène, indométhacine, indoprofène, kétoprofène, lonazolac, loxoprofène, acide méclofénamique, acide méfanamique, naproxène, acides proprioniques, acides salicyliques, sulindac, tolmétine, méloxicam, oxicams, piroxicam, tenoxicam, étodolac et oxaprozine.

11. Dispositif selon l'une quelconque des revendications 1 à 10, sous forme d'un film.

12. Dispositif selon la revendication 11, dans lequel ladite protéine soluble dans l'eau est choisie dans le groupe constitué de gélatine, collagène, albumine, une enzyme et le fibrinogène.

13. Dispositif selon l'une quelconque des revendications 1 à 12, dans lequel ledit polymère est réticulé par un procédé de traitement en présence d'un agent de réticulation, dans lequel ledit procédé de traitement est choisi dans le groupe constitué de chaleur, humidité, pression, radiation et vapeurs d'un agent de réticulation, et dans lequel la réticulation rend ladite protéine insoluble dans l'eau tout en permettant la libération graduelle des agents actifs à partir dudit système.

14. Dispositif selon la revendication 6, dans lequel ledit agent plastifiant est choisi dans le groupe constitué de dérivés de glycols, phthalates, dérivés de citrate, benzoates, esters butyliques ou glycoliques d'acides gras, huiles minérales raffinées, camphre, acide oléique, huile de ricin, huile de maïs et alcools de sucres.

15. Dispositif selon l'une quelconque des revendications 1 à 14, dans lequel ledit agent antibactérien est choisi dans le groupe constitué de pénicilline, céphalosporine, tétracycline, oxytétracycline, chlortétracycline, métronidazole, chloramphénicol, streptomycine, néomycine, une sulfonamide, un composé phénolique, un mercurial, un composé ammonium quaternaire, doxycycline et chlorhexidine, ou des sels de ceux-ci.

16. Dispositif à libération orale selon la revendication 1 pour le traitement de maladies parodontales, ledit dispositif étant sous forme de dose unitaire unique solide pour une administration dans la poche parodontale d'un patient pour se transformer dans ladite poche en une forme molle, le système comprenant, sur une base pondérale, entre environ 5 et environ 25% de flurbiprofène, entre environ 15 et environ 35% de chlorhexidine di-gluconate, entre environ 30 et environ 50% de gélatine hydrolysée, entre environ 2 et environ 7,5% d'agent de réticulation, entre environ 0,5 et environ 15% de tensioactif et entre environ 5 et environ 15% de plastifiant.

17. Implant parodontal comprenant un dispositif selon l'une quelconque des revendications 1 à 16.

18. Utilisation de:
(i) un polymère biodégradable ou bioérodable acceptable sur le plan pharmaceutique ;
(ii) une quantité efficace sur le plan thérapeutique d'au moins un agent antibactérien ;
(iii) une quantité efficace sur le plan thérapeutique d'au moins un agent anti-inflammatoire, le rapport de poids relatif entre l'agent antibactérien et l'agent anti-inflammatoire allant de 7:1 environ à 1:5 environ; et
(iv) un tensioactif;
pour la préparation d'une composition pharmaceutique pour une utilisation dans le traitement de problèmes dentaires.
